(19) Europäisches Patentamt
European Patent Office
Office européen des brevets

(11) Veröffentlichungsnummer: **0 010 723**
**B1**

(12) # EUROPÄISCHE PATENTSCHRIFT

(45) Veröffentlichungstag der Patentschrift: **29.07.81**

(21) Anmeldenummer: **79104103.1**

(22) Anmeldetag: **23.10.79**

(51) Int. Cl.³: **C 07 D233/56**, C 07 D233/58, C 07 D233/60, C 07 D233/61, C 07 D233/92, C 07 D233/94, C 07 D233/95, A 01 N 43/50

(54) **Imidazol-Kupferkomplexverbindungen, Verfahren zu ihrer Herstellung und diese enthaltende Fungizide.**

(30) Priorität: **02.11.78 DE 2847441**

(43) Veröffentlichungstag der Anmeldung: **14.05.80 (Patentblatt 80/10)**

(45) Bekanntmachung des Hinweises auf die Patenterteilung: **29.07.81 Patentblatt 81/30**

(84) Benannte Vertragsstaaten: **AT BE CH DE FR GB IT LU NL SE**

(56) Entgegenhaltungen: **DE - A - 2 604 047**

(73) Patentinhaber: **BASF Aktiengesellschaft**
**Carl-Bosch-Strasse 38**
**D-6700 Ludwigshafen (DE)**

(72) Erfinder: **Dockner, Toni, Dr.**
**Grossgasse 6**
**D-6701 Meckenheim (DE)**
Erfinder: **Frank, Anton**
**Bannwasserstrasse 72**
**D-6700 Ludwigshafen (DE)**
Erfinder: **Pommer, Ernst-Heinrich, Dr.**
**Berliner Platz 7**
**D-6703 Limburgerhof (DE)**

EP 0 010 723 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Imidazol-Kupferkomplexverbindungen, Verfahren zu ihrer Herstellung und diese enthaltende Fungizide

Die vorliegende Erfindung betrifft wertvolle neue Imidazol-Kupferkomplexverbindungen mit guter fungizider Wirkung, Fungizide, die diese Verbindungen als Wirkstoffe enthalten, Verfahren zu ihrer Herstellung und ihre Verwendung zur Bekämpfung von Pilzen.

Es ist bekannt, Kupferoxychlorid als Fungizid zu verwenden. Seine Wirkung ist jedoch unbefriedigend.

Aus der DE-OS 26 04 047 sind fungizide Kupferkomplexverbindungen von substituierten Imidazolen bekannt, wobei das Imidazol durch einen Dichlorphenyl- oder Phenylhexylen- oder -heptylenrest substituiert ist. Hierdurch werden die neuen fungizidwirksamen Verbindungen, in denen das Imidazol durch den Benzylrest oder Phenyläthylrest substituiert ist, nicht nahegelegt.

Es wurde gefunden, daß Imidazol-Kupferkomplexverbindungen der Formel

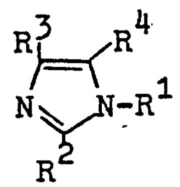 $.Cu(X)_2$

wobei $R^1$ Wasserstoff, einen Alkylrest mit 1 bis 18 Kohlenstoffatomen, der gegebenenfalls substituiert ist durch Morpholinyl, Piperidinyl, Piperazinyl, Pyrrolidinyl, Hydroxyl oder Amino, das selbst wieder gegebenenfalls substituiert ist durch Phenyl und Benzyl oder durch zwei Alkylreste mit 1 oder 4 Kohlenstoffatomen je Rest bedeuten, $R^1$ ferner Benzyl, Cyanobenzyl, Phenyläthyl, Phenylchloräthyl, Isobutylbenzyl oder einen Phenylrest, der gegebenenfalls substituiert ist durch Amino bedeutet, $R^2$ Wasserstoff, einen Alkylrest mit 1 bis 18 Kohlenstoffatomen, der gegebenenfalls substituiert ist durch den Piperonylrest oder Pyrrolidinrest oder den Acetoxyrest oder den Hydroxyrest oder den Phenylrest, der selbst wieder gegebenenfalls substituiert ist durch Alkyl mit 4 C-Atomen bedeutet, $R^2$ ferner einen Alkenylrest mit 9 Kohlenstoffatomen oder den Imidazolylrest oder den Indanylrest oder einen Phenylrest, der einfach substituiert ist durch den Methoxyrest oder den Hydroxyrest oder zweifach substituiert ist durch den Methylrest bedeutet, $R^3$ Wasserstoff, einen Alkylrest mit 1 bis 4 Kohlenstoffatomen oder den Carboxylrest oder den Cyanrest oder den Nitrorest, $R^4$ Wasserstoff, einen Alkylrest mit 1 bis 3 Kohlenstoffatomen, der Cyanrest, den Benzylrest, den Nitrorest, oder den Cyclohexylrest und X das Anion einer Mineral- oder niederen Fettsäure bedeuten, eine bessere fungizide Wirkung gegenüber Pilzen insbesondere phytopathogenen Pilzen z.B. Plasmopara viticola an Reben und Phytophthora infestans an Tomaten zeigen als bekannte Fungizide.

Die neuen Kupferkomplexverbindungen sind teils kristalline, teils pastenförmige oder ölige Substanzen die in Wasser, Alkoholen und verschiedenen organischen Lösungsmitteln löslich sind.

Sie werden hergestellt indem man Imidazole der Formel

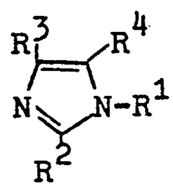

in der $R^1$-$R^4$ die im Anspruch 1 angeführten Bedeutungen hatten, mit einem neutralen oder basischen Kupfersalz einer Mineralsäure oder niederen Fettsäure umsetzt. Für die Umsetzung wird bevorzugt Kupfer-II-chlorid oder ein anderes Kupfersalz einer Mineralsäure oder niederen Fettsäure verwendet. Man kann beispielsweise das Imidazol mit dem Kupfersalz vermischen oder die methanolische Lösung des Imidazols und z.B. Kupferchlorid mischen und danach das Methanol abdestillieren.

In der allgemeinen Formel der neuen Imidazol-Kupferkomplexverbindungen bedeuten

$R^1$ beispielsweise: Wasserstoff, Methyl-, Äthyl-, Propyl-, Hexyl-, Heptyl-, Oktyl-, Decyl-, Undecyl-, Dodecyl-, Tridecyl-, Tetradecyl-, Hexadecyl-, Oktadecyl-, Phenyl-, p-Aminophenyl-, Benzyl-, Cyanobenzyl-, Phenyläthyl-, Isobutylbenzyl-, Pyrrolidyläthyl-, Morpholinoäthyl-, Piperidinoäthyl-, Dibutylaminoäthyl-, Phenylbenzylaminoäthyl-,

$R^2$ bedeutet beispielsweise: Wasserstoff, Methyl-, Äthyl-, Propyl-, Isopropyl-, Oktyl-, Decyl-, Dodecyl-, Oktadecyl-, Phenyl-, Methoxyphenyl-, Benzyl-, Indanyl-, Dimethylphenyl-, Butylphenyl-,

$R^3$ bedeutet beispielsweise: Wasserstoff, Methyl-, Äthyl-, Propyl-, Isopropyl-, Butyl-, Isobutyl-, Carboxyl-, den Cyanrest oder den Nitrorest,

$R^4$ bedeutet beispielsweise: Wasserstoff, die Nitrogruppe, Cyano-, Benzyl-, Cyclohexyl-gruppe.

Mineralsäuren sind beispielsweise Salzsäure, Bromwasserstoffsäure, Schwefelsäure, Salpetersäure, Phosphorsäure, und Sulfonsäuren z.B. p-Toluolsulfonsäure, Dodecylbenzolsulfonsäure. Nieder Fettsäuren sind beispielsweise : Ameisensäure, Essigsäure, Propionsäure, Oxalsäure, Weinsäure, Asorbinsäure, Zitronensäure.

Die Herstellung der Imidazol-Kupferkomplexverbindungen durch Umsetzung eines Imidazols mit einem Kupfersalz kann beispielsweise ohne Lösungsmittel oder in Gegenwart eines Lösungsmittels bei Temperaturen von − 10 bis + 170 °C erfolgen.

Lösungsmittel sind beispielsweise Wasser, Methanol, Ethanol, Isopropanol, Dimethylformamid. Auch ein flüssiges Imidazol selbst kann als Lösungsmittel für die Umsetzung dienen. Die Ausgangsprodukte werden in stöchiometrischen Mengen oder im Überschuß eines Ausgangsproduktes angewendet. Da die Kupfersalze leicht zugänglich sind, werden sie bevorzugt im Überschuß beispielsweise im bis zu 10 fachen Überschuß über die stöchiometrischen Mengen hinaus angewendet. Die Umsetzungen laufen bei Normaldruck ab. Selbstverständlich kann auch bei Über- oder Unterdruck umgesetzt werden. Dies ist aber nicht notwendig. Daher wird die Umsetzung bei Normaldruck unter Verwendung von Methanol als Lösungsmittel mit geringem Überschuß (bis zu 10 %) an Kupferchlorid bei einer Temperatur von 20 °C bevorzugt. Das folgende Beispiel erläutert die Umsetzung.

Beispiel 1

Herstellung von 1-Dodecylimidazol-$CuCl_2$

Eine Lösung von 42,5 Teilen (Gewichtsteilen) $CuCl_2 . 2H_2O$ in 200 Teilen Methanol wird unter Kühlung mit 59 Teilen 1-Dodecylimidazol versetzt. Die Mischung wird im Vakuum eingedampft. Man erhält 93 Teile eines dunklen, syrupösen Rückstands der 16,0 % Cu enthält (Wirkstoff 1).

Beispiel 2

Entsprechend Beispiel 1 werden durch Umsetzung verschiedener Imidazole mit $CuCl_2$ die folgende Verbindungen enthalten.

| Wirkstoff | | |
|---|---|---|
| 2 | Imidazol. $CuCl_2$ (gelbgrüne Kistalle) | Fp = 198 °C |
| 3 | 1-Hexylimidazol. $CuCl_2$ dunkles Öl | |
| 4 | 1-(2-pyrrolidyläthyl)-imidazol. $CuCl_2$ | Fp = 119 °C |
| 5 | 1-(2-morpholinoäthyl)-imidazol. $CuCl_2$ | Fp = 108 °C |
| 6 | 1-(2-piperidinoäthyl)-imidazol. $CuCl_2$ | Fp = 106 °C |
| 7 | 1-Heptylimidazol. $CuCl_2$ dunkles Öl | |
| 8 | 1-(2-Cyanobenzyl)-imidazol. $CuCl_2$ | Fp = 209-210 °C |
| 9 | 1-(1-Phenyl-2-chloräthyl)-imidazol. $CuCl_2$ | Fp = 163- 64 °C |
| 10 | 1-Oktylimidazol. $CuCl_2$ dunkles Öl | |
| 11 | 1-(2-Äthylhexyl)-imidazol. $CuCl_2$ Paste | |
| 12 | 1-Decylimidazol. $CuCl_2$ dunkles Öl | |
| 13 | 1-(2-Dibutylaminoäthyl)-imidazol. $CuCl_2$ | Fp = 128 °C |
| 14 | 1-(p-Isobutylbenzyl)-imidazol. $CuCl_2$ | Fp = 64- 65 °C |
| 15 | 1-Undecylimidazol. $CuCl_2$ Öl | |
| 16 | 1-Isotridecylimidazol. $CuCl_2$ Öl | |
| 17 | 1-Tetradecylimidazol. $CuCl_2$ | Fp = 74 °C |
| 18 | 1-(2-N-Phenyl-N-benzylaminoäthyl)-imidazol. $CuCl_2$ | Fp = 60 °C |
| 19 | 1-Hexadecylimidazol. $CuCl_2$ | Fp = 68- 70 °C |
| 20 | 1-Oktadecylimidazol. $CuCl_2$ | Fp = 54- 55 °C |
| 21 | 1-(p-Aminophenyl)-imidazol. $CuCl_2$ | Fp = 208 °C |
| 22 | 2-Heptylimidazol. $CuCl_2$ | Fp = 115 °C |
| 23 | 2-(2,6-Dimethylhepten-6-yl)-imidazol. $CuCl_2$ Öl | |
| 24 | 2-(1-Piperonylpropyl)-imidazol. $CuCl_2$ Öl | |
| 25 | 2-(1-p-Isobutylphenyläthyl)-imidazol. $CuCl_2$ Öl | |
| 26 | 2-Dodecylimidazol. $CuCl_2$ Paste | |
| 27 | 2-Oktadecylimidazol. $CuCl_2$ Paste | |
| 28 | 2,2'-Diimidazolyl. $CuCl_2$ | Fp = 360 °C |
| 29 | 2-Phenylimidazol. $CuCl_2$ | Fp = 170 °C |
| 30 | 2-(2-Methoxyphenyl)-imidazol. $CuCl_2$ | Fp = 107 °C |
| 31 | 1,2-Dimethylimidazol. $CuCl_2$ | Fp = 225-226 °C |
| 32 | 1-Methyl-2-äthylimidazol. $CuCl_2$ | Fp = 237-239 °C |
| 33 | 1-(2-Dimethylaminoäthyl)-2-methylimidazol. $CuCl_2$ | Fp = 116 °C |
| 34 | 1-(2-Piperazinoäthyl)-2-methylimidazol. $CuCl_2$ | Fp = 135-139 °C |
| 35 | 1-(2-Pyrrolidinoäthyl)-2-methylimidazol. $CuCl_2$ | Fp = 120-127 °C |
| 36 | 1-Methyl-2-(2-Pyrrolidinoäthyl)-imidazol. $CuCl_2$ | Fp = 134-135 °C |
| 37 | 1-(3,3-dimethylbutyl)-2-methylimidazol. $CuCl_2$ | Fp = 240 °C |

| | | |
|---|---|---|
| 38 | 1-Methyl-2-oktylimidazol. $CuCl_2$ Paste | |
| 39 | 1-Decyl-2-methylimidazol. $CuCl_2$ | Fp = 130-132 °C |
| 40 | 1-(1-Methyl-2-hydroxyäthyl)-2-indanylimidazol. $CuCl_2$ | Fp = 79- 80 °C |
| 41 | 1-Dodecyl-2-methylimidazol. $CuCl_2$ | Fp = 154-155 °C |
| 42 | 1-Phenyl-2-(3,4-dimethylphenyl)-imidazol. $CuCl_2$ | Fp = 180-181 °C |
| 43 | 1-Dodecyl-2-(1,1-dimethyl-2-acetoxiäthyl)-imidazol. $CuCl_2$ Öl | |
| 44 | 1-(2-N-Phenyl-N-benzylaminoäthyl)-2-phenylimidazol. $CuCl_2$ | Fp = 69- 70 °C |
| 45 | 1-Dodecyl-2-(1-p-tertiär Butylphenyläthyl)-imidazol. $CuCl_2$ | Fp = 114-116 °C |
| 46 | 2-Methyl-4(5)-nitroimidazol. $CuCl_2$ | Fp = 238 °C |
| 47 | 1,2-Dimethyl-5-nitroimidazol. $CuCl_2$ | Fp = 204 °C |
| 48 | 1-(2-Aminoäthyl)-2-methyl-5-nitroimidazol. $CuCl_2$ | Fp = 90- 91 °C |
| 49 | 1-Methyl-2-isopropyl-5-nitroimidazol. $CuCl_2$ | Fp = 242 °C |
| 50 | 2,4(5)-Dimethyl-5(4)-propylimidazol. $CuCl_2$ Öl | |
| 51 | 2-Phenyl-4,5-dimethylimidazol. $CuCl_2$ | Fp = 161 °C |
| 52 | 2-(2-Hydroxyphenyl)-4,5-dimethylimidazol. $CuCl_2$ Paste | |
| 53 | 2-(2-Phenylpropyl)-4(5)-methyl-5(4)-isobutylimidazol. $CuCl_2$ | Fp = 60 °C |
| 54 | 1-Dodecyl-4(5)-cyano-5(4)-carboxylimidazol. $CuCl_2$ | Fp = 214 °C |
| 55 | 1,2,4-Trimethyl-5-nitroimidazol. $CuCl_2$ Paste | |
| 56 | 1,4-Dimethyl-2-hydroxymethyl-5-nitroimidazol. $CuCl_2$ | Fp = 193 °C |
| 57 | 1,2,4,5-Tetramethylimidazol. $CuCl_2$ Paste | |
| 58 | 1-(2-Dimethylaminoäthyl)-2,4,5-trimethylimidazol. $CuCl_2$ Öl | |
| 59 | 1-(2-Pyrrolidyläthyl)-2,4,5-trimethylimidazol. $CuCl_2$ | Fp = 105 °C |
| 60 | 1-Dodecyl-2,4,5-trimethylimidazol. $CuCl_2$ Öl | |
| 61 | 1-Dodecyl-2,4-diäthyl-5-methylimidazol. $CuCl_2$ Öl | |
| 62 | 1-Dodecyl-2-phenyl-4,5-dimethylimidazol. $CuCl_2$ Öl | |
| 63 | 1-Dodecyl-2-methyl-4-äthyl-5-cyclohexylimidazol. $CuCl_2$ Öl | |
| 64 | 1-Dodecyl-2-methyl-4-äthyl-5-benzylimidazol. $CuCl_2$ Öl | |

Beispiel 3

118 Teile 1-Dodecylimidazol und 80 Teile wasserfreies Kupfersulfat werden im Rührkolben auf 100° erhitzt wobei unter schwach exothermer Reaktion eine homogene Schmelze entsteht, die nach kurzer Zeit erstarrt. Man erhält 168 Teile 1-Dodecyl-imidazol-$CuSO_4$-Komplex vom Schmelzpunkt 216-218 °C (Wirkstoff 65).

Beispiel 4

104 Teile 1-Decylimidazol und 100 Teile Kupfer-II-acetat Monohydrat werden bei 140° innig vermischt. Nach dem Erkalten erhält man 195 Teile einer hellblauen, pastenförmigen Masse von 1-Decylimidazol-Kupferacetat-Komplex (Wirkstoff 66).

Die erfindungsgemäßen Wirkstoffe zeigen eine starke fungitoxische Wirkung gegenüber phytopathogenen Pilzen, insbesondere aus der Pilzklasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Pseudoperonospora humuli an Hopfen, Peronospora tabacina an Tabak sowie Plasmopara viticola an Reben. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha.

Darüber hinaus lassen sich mit den neuen Verbindungen auch Pilze, die Keimlingskrankheiten hervorrufen, beispielsweise Pythium-Arten, bekämpfen.

Einige der Verbindungen sind auch bakterizid und algizid wirksam.

Die erfindungsgemäßen Substanzen können in die üblichen Formulierungen übergeführt werden, wie Lösungen, Emulsionen, Suspensionen, Stäube, Pulver, Pasten und Granulate.

Die Anwendungsformen richten sich ganz nach den Verwendungszwecken, sie sollen in jedem Fall eine feine gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel wie nichtionogene und anionische Emulgatoren (z.B. Polyoxyäthylen-Fettalkohol-Äther, Alkylsulfonate und Arylsulfonate) und Dispergiermittel, wie Lignin-, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Metallkomplexen kombiniert werden können, sind beispielsweise :

Dithiocarbamate und deren Derivate, wie Ferridimethyldithiocarbamat, Zinkdimethyldithiocarbamat, Manganäthylenbisdithiocarbamat, Mangan-Zink-äthylendiamin-bis-dithiocarbamat, Zinkäthylenbisdithiocarbamat, Tetramethylthiuramdisulfide, Ammoniak-Komplex von Zink-(N,N-äthylen-bis-dithiocarbamat) und Zink-(N,N-propylen-bis-dithiocarbamat), Ammoniak-Komplex von Zink-(N,N'-propylen-bis-dithiocarbamat) und N,N'-Polypropylen-bis-(hiocarbamoyl)-disulfid ;

Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat ;

heterocyclische Strukturen, wie N-Trichlormethylthio-tetrahydrophthalimid, N-Trichlormethylthiophthalimid, 2-Heptadecyl-2-imidazolin-acetat, 2,4-Dichlor-6-(o-chloranilino)-s-triazin, 0,0-Diäthyl-phthalimidophonothioat, 5-Amino-1-(bis-(dimethylamino)-phosphinyl)-3-phenyl-1,2,4-triazol, 5-Äthoxy-3-trichloromethyl-1,2,4-thiadiazol, 2,3-Dicyano-1,4-dithiaanthrachinon, 2-Thio-1,3-dithio-(4,5-b)-chinoxalin, 1-(Butylcarbamoyl)-2-benzimidazol-carbaminsäuremethylester, 2-Methoxycarbonylamino-benzimidazol, 2-Rhodanmethylthio-benzthiazol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin-4,4-dioxid 2,3-Dihydro-5-carboxanilido-6-methyl-1,4-oxathiin, 2-(Furyl-(2))-benzimidazol, Piperazin-1,4-diylbis-(1-(2,2,2-trichlor-äthyl)-formamid, 2-(Thiazolyl-(4))-benzimidazol, 5-Butyl-2-dimethylamino-4-hydroxy-6-methyl-pyrimidin, Bis-(p-chlorphenyl)-3-pyridinmethanol, 1,2-Bis-(3-äthoxycarbonyl-2-thioureido)-benzol, 1,2-Bis-(3-methoxycarbonyl-2-thioureido)-benzol und verschiedene Fungizide, wie Dodecylguanidinacetat, 3-(3-(3,5-Dimethyl-2-oxycyclohexyl)-2-hydroxyäthyl)-glutarimid, Hexachlorbenzol, N-Dichlorfluormethylthio-N',N'-dimethyl-N-phenyl-schwefelsäure-diamid, 2,5-Dimethyl-furan-3-carbonsäureanilid, 2,5-Dimethyl-furan-3-carbonsäure-cyclohexylamid, 2-Methyl-benzoesäure-anilid, 2-Jod-benzoesäure-anilid, 1-(3,4-Dichloranilino)-1-formylamino-2,2,2-trichloräthan, 2,6-Dimethyl-N-tridecyl-morpholin bzw. dessen Salze, 2,6-Dimethyl-N-cyclododecyl-morpholin bzw. dessen Salze.

Die folgenden Beispiele erläutern die fungizide Wirkung.

## Beispiel 5

Fungizide Wirksamkeit gegen Phytophthora infestans an Tomaten

Blätter von Tomatenpflanzen der Sorte « Professor Rudloff » werden mit wäßrigen Suspensionen, die 80 % (Gewichtsprozent) des zu prüfenden Wirkstoffes und 20 % Natriumligninsulfonat in der Trockensubstanz enthalten, besprüht. Es werden 0,2 und 0,1 %ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann :

0 = kein Pilzbefall, abgestuft bis 5 = Totalbefall (Kontrolle)

| Wirkstoff | Befall der Blätter nach Spritzung mit ... %iger Wirkstoffbrühe | |
|---|---|---|
| | 0,2 | 0,1 |
| 1 | 0 | 0 |
| 4 | 0 | 0 |
| 5 | 0 | 0 |
| 7 | 0 | 0 |
| 8 | 0 | 0 |

| | | |
|---|---|---|
| 10 | 0 | 0 |
| 11 | 0 | 0 |
| 12 | 0 | 0 |
| 13 | 0 | 0 |
| 14 | 0 | 0 |
| 18 | 0 | 0 |
| 20 | 0 | 0 |
| 22 | 0 | 0 |
| 32 | 0 | 0 |
| 33 | 0 | 0 |
| 38 | 0 | 0 |
| 39 | 0 | 0 |
| 42 | 0 | 0 |
| 43 | 0 | 0 |
| 59 | 0 | 0 |
| 60 | 0 | 0 |
| 62 | 0 | 0 |
| 63 | 0 | 0 |
| 66 | 0 | 0 |
| Kupferoxychlorid (bekannt) | 1 | 2 |
| Kontrolle (unbehandelt) | 5 | |

Beispiel 6

Fungizide Wirksamkeit gegen Plasmopara viticola an Reben

Blätter von Topfreben der Sorte Müller-Thurgau werden mit wäßrigen Suspensionen, die 80 % (Gewichtsprozent) des zu prüfenden Wirkstoffes und 20 % Natriumligninsulfonat in der Trockensubstanz enthalten, besprüht. Es werden 0,1, 0,05 und 0,025 %ige Spritzbrühen (bezogen auf die Trockensubstanz) verwendet. Nach dem Abtrocknen des Spritzbelages werden die Blätter mit einer Zoosporenaufschwemmung von Plasmopara viticola infiziert. Die Pflanzen kommen dann zuerst für 16 Stunden in eine wasserdampfgesättigte (feuchte) Kammer bei 20 °C und anschließend 8 Tage in ein Gewächshaus mit Temperaturen zwischen 20 und 30 °C. Nach dieser Zeit werden die Pflanzen zur Beschleunigung und Verstärkung des Sporangienträgerausbruches abermals während 16 Stunden in der feuchten Kammer aufgestellt. Dann wird eine Beurteilung des Krankheitsausbruches vorgenommen; hierbei bedeuten 0 = kein Pilzbefall, abgestuft bis 5 = Totalbefall (Kontrolle).

| Wirkstoff | Befall der Blätter nach Spritzung mit ... %iger Wirkstoffbrühe | | |
|---|---|---|---|
| | 0,1 | 0,05 | 0,025 |
| 10 | 0 | 0 | 0 |
| 14 | 0 | 0 | 1 |
| 32 | 0 | 0 | 0 |

| | | | |
|---|---|---|---|
| 38 | 0 | 0 | 0 |
| 39 | 0 | 0 | 2 |
| 42 | 0 | 0 | 0 |
| 43 | 0 | 0 | 2 |
| Kupferoxychlorid (bekannt) | 2 | 3 | 4 |
| Kontrolle (unbehandelt) | | 5 | |

Beispiel 7

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 8

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 10 000 Gewichtsteilen Wasser erhält man eine wäßrige Dispersion, die 0,02 Gewichtsprozent des Wirkstoffs enthält.

Beispiel 9

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 10

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 11

20 Gewichtsteile des Wirkstoffs werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 12

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 13

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 14

40 Gewichtsteile des Wirkstoffs 1 werden mit 10 Teilen Natriumsalz eines Phenolsulfonsäure-

harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 15

20 Teile des Wirkstoffs 2 werden mit 2 Teilen Calciumsalz der Dodecylbenzolsulfonsäure, 8 Teilen Fettalkoholpolyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Ansprüche** (für Vertragsstaaten : BE, CH, DE, FR, GB, IT, LU, NL, SE)

1. Imidazol-Kupferkomplexverbindungen der Formel

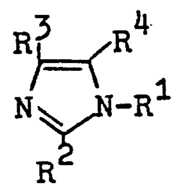 $\cdot Cu(X)_2$

wobei $R^1$ bedeutet Wasserstoff, einen Alkylrest mit 1 bis 18 Kohlenstoffatomen, der gegebenenfalls substituiert ist durch Morpholinyl, Piperidinyl, Piperazinyl, Pyrrolidinyl, Hydroxyl oder Amino, das selbst wieder gegebenenfalls substituiert ist durch Phenyl und Benzyl oder durch zwei Alkylreste mit 1 oder 4 Kohlenstoffatomen je Rest. $R^1$ bedeutet ferner Benzyl, Cyanobenzyl, Phenyläthyl, Phenyl-chloräthyl, Isobutylbenzyl oder einen Phenylrest, der gegebenenfalls substituiert ist durch Amino, $R^2$ bedeutet Wasserstoff, einen Alkylrest mit 1 bis 18 Kohlenstoffatomen, der gegebenenfalls substituiert ist durch den Piperonylrest oder Pyrrolidinrest oder den Acetoxyrest oder den Hydroxyrest oder den Phenylrest, der selbst wieder ggf. substituiert ist durch Alkyl mit 4 C-Atomen. $R^2$ bedeutet ferner einen Alkenylrest mit 9 Kohlenstoffatomen oder den Imidazolylrest oder den Indanylrest oder einen Phenylrest, der einfach substituiert ist durch den Methoxyrest oder den Hydroxyrest oder zweifach substituiert ist durch den Methylrest. $R^3$ bedeutet Wasserstoff, einen Alkylrest mit 1 bis 4 Kohlenstoffatomen oder den Carboxylrest oder den Cyanrest oder den Nitrorest und $R^4$ bedeutet Wasserstoff, einen Alkylrest mit 1 bis 3 Kohlenstoffatomen, der Cyanrest, den Benzylrest, den Nitrorest oder den Cyclohexylrest. X bedeutet das Anion einer Mineral- oder niederen Fettsäure.

2. Fungizid, enthaltend eine Imidazol-Kupferkomplexverbindung gemäß Anspruch 1.

3. Fungizid, enthaltend einen festen oder flüssigen Trägerstoff und eine Imidazol-Kupferkomplexverbindung gemäß Anspruch 1.

4. Verfahren zur Herstellung eines Fungizids, *dadurch gekennzeichnet,* daß man einen festen oder flüssigen Trägerstoff vermischt mit einer Imidazol-Kupferkomplex-Verbindung gemäß Anspruch 1.

5. Verfahren zur Bekämpfung von Pilzen, *dadurch gekennzeichnet,* daß man die Pilze oder die vor Pilzbefall zu schützenden Gegenstände behandelt mit einer Imidazol-Kupferkomplexverbindung gemäß Anspruch 1.

6. Verfahren zur Herstellung einer Imidazol-Kupferkomplexverbindung gemäß Anspruch 1, *dadurch gekennzeichnet,* daß man ein Imidazol der Formel

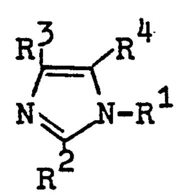

in der $R^1$ bis $R^4$ die im Anspruch 1 angeführten Bedeutungen haben, mit einem neutralen oder basischen Kupfersalz einer Mineralsäure oder niederen Fettsäure umsetzt.

7. Imidazol-Kupferkomplexverbindungen ausgewählt aus der Gruppe bestehend aus 1-Oktylimidazol-Cu-$Cl_2$, 1-Dodecylimidazol-$CuCl_2$, 1-Tetradecylimidazol-$CuCl_2$.

8. Fungizid, enthaltend eine Imidazol-Kupferkomplexverbindung, ausgewählt aus der Gruppe, bestehend aus 1-Oktylimidazol-Cu-$Cl_2$, 1-Dodecylimidazol-$CuCl_2$, 1-Tetradecylimidazol-$CuCl_2$.

**Ansprüche** (für den Vertragsstaat : AT)

1. Fungizid, enthaltend eine Imidazol-Kupferkomplexverbindung der Formel

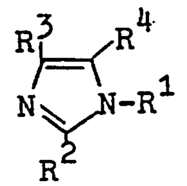

$\cdot Cu(X)_2$

wobei $R^1$ bedeutet Wasserstoff, einen Alkylrest mit 1 bis 18 Kohlenstoffatomen, der gegebenenfalls substituiert ist durch Morpholinyl, Piperidinyl, Piperazinyl, Pyrrolidinyl, Hydroxyl oder Amino, das selbst wieder gegebenenfalls substituiert ist durch Phenyl und Benzyl oder durch zwei Alkylreste mit 1 oder 4 Kohlenstoffatomen je Rest. $R^1$ bedeutet ferner Benzyl, Cyanobenzyl, Phenyläthyl, Phenylchloräthyl, Isobutylbenzyl oder einen Phenylrest, der gegebenenfalls substituiert ist durch Amino, $R^2$ bedeutet Wasserstoff, einen Alkylrest mit 1 bis 18 Kohlenstoffatomen, der gegebenenfalls substituiert ist durch den Piperonylrest oder Pyrrolidinrest oder den Acetoxyrest oder den Hydroxyrest oder den Phenylrest, der selbst wieder ggf. substituiert ist durch Alkyl mit 4 C-Atomen. $R^2$ bedeutet ferner einen Alkenylrest mit 9 Kohlenstoffatomen oder den Imidazolylrest oder den Indanylrest oder einen Phenylrest, der einfach substituiert ist durch den Methoxyrest oder den Hydroxyrest oder zweifach substituiert ist durch den Methylrest. $R^3$ bedeutet Wasserstoff, einen Alkylrest mit 1 bis 4 Kohlenstoffatomen oder den Carboxylrest oder den Cyanrest oder den Nitrorest und $R^4$ bedeutet Wasserstoff, einen Alkylrest mit 1 bis 3 Kohlenstoffatomen, den Cyanrest, den Benzylrest, den Nitrorest oder den Cyclohexylrest X bedeutet das Anion einer Mineral- oder niederen Fettsäure.

2. Fungizid, enthaltend einen festen oder flüssigen Trägerstoff und eine Imidazol-Kupferkomplexverbindung wie in Anspruch 1 definiert.

3. Verfahren zur Herstellung eines Fungizids gemäß Anspruch 2, *dadurch gekennzeichnet,* daß man einen festen oder flüssigen Trägerstoff vermischt mit einer Imidazol-Kupferkomplex-Verbindung wie in Anspruch 1 definiert.

4. Verfahren zur Bekämpfung von Pilzen, *dadurch gekennzeichnet,* daß man die Pilze oder die vor Pilzbefall zu schützenden Gegenstände behandelt mit einem Fungizid gemäß Anspruch 1.

5. Verfahren zur Herstellung einer Imidazol-Kupferkomplexverbindung für ein Fungizid gemäß Anspruch 1, *dadurch gekennzeichnet,* daß man ein Imidazol der Formel

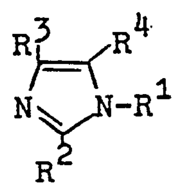

in der $R^1$-$R^4$ die im Anspruch 1 angeführten Bedeutungen haben, mit einem neutralen oder basischen Kupfersalz einer Mineralsäure oder niederen Fettsäure umsetzt.

6. Fungizid, enthaltend eine Imidazol-Kupferkomplexverbindung, ausgewählt aus der Gruppe, bestehend aus 1-Oktylimidazol-Cu-$Cl_2$, 1-Dodecylimidazol-$CuCl_2$, 1-Tetradecylimidazol-$CuCl_2$.

**Claims** (for the Contracting States : BE, CH, DE, FR, GB, IT, LU, NL, SE)

1. Imidazole-copper complex compounds of the formula

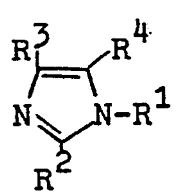

$\cdot Cu(X)_2$

where $R^1$ denotes hydrogen, alkyl of 1 to 18 carbon atoms optionally substituted by morpholinyl, piperidinyl, piperazinyl, pyrrolidinyl, hydroxyl or amino which in its turn is optionally substituted by phenyl and benzyl or by two alkyls of 1 or 4 carbon atoms per radical, $R^1$ further denotes benzyl, cyanobenzyl, phenylethyl, phenylchloroethyl, isobutylbenzyl or phenyl optionally substituted by amino, $R^2$ denotes hydrogen, alkyl of 1 to 18 carbon atoms optionally substituted by piperonyl or pyrrolidinyl, or acetoxy,

hydroxy or phenyl which in its turn is optionally substituted by alkyl of 4 carbon atoms, $R^2$ further denotes alkenyl of 9 carbon atoms, or imidazolyl, indanyl or phenyl which is monosubstituted by methoxy or hydroxy, or disubstituted by methyl, $R^3$ denotes hydrogen, alkyl of 1 to 4 carbon atoms, carboxyl, cyano or nitro, $R^4$ denotes hydrogen, alkyl of 1 to 3 carbon atoms, cyano, benzyl, nitro or cyclohexyl, and X denotes the anion of a mineral acid or lower fatty acid.

2. A fungicide containing an imidazole-copper complex compound as claimed in claim 1.

3. A fungicide containing a solid or liquid carrier and an imidazole-copper complex compound as claimed in claim 1.

4. A process for the preparation of a fungicide, *characterized in that* a solid or liquid carrier is mixed with an imidazole-copper complex compound as claimed in claim 1.

5. A process for combating fungi, *characterized in that* the fungi or the objects to be protected against fungus attack are treated with an imidazole-copper complex compound as claimed in claim 1.

6. A process for the manufacture of an imidazole-copper complex compound as claimed in claim 1, *characterized in that* an imidazole of the formula

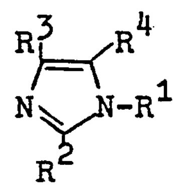

where $R^1$ to $R^4$ have the meanings given in claim 1, is reacted with a neutral or basic copper salt of a mineral acid or lower fatty acid.

7. An imidazole-copper complex compound selected from the group consisting of 1-octylimidazole-$CuCl_2$, 1-dodecylimidazole-$CuCl_2$ and 1-tetradecylimidazole-$CuCl_2$.

8. A fungicide containing an imidazole-copper complex compound selected from the group consisting of 1-octylimidazole-$CuCl_2$, 1-dodecylimidazole-$CuCl_2$ and 1-tetradecylimidazole-$CuCl_2$.

**Claims** (for the Contracting State : AT)

1. A fungicide containing an imidazole-copper complex compound of the formula

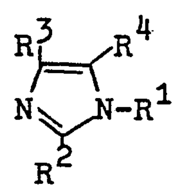 . $Cu(X)_2$

where $R^1$ denotes hydrogen, alkyl of 1 to 18 carbon atoms optionally substituted by morpholinyl, piperidinyl, piperazinyl, pyrrolidinyl, hydroxyl or amino which in its turn is optionally substituted by phenyl and benzyl or by two alkyls of 1 or 4 carbon atoms per radical, $R^1$ further denotes benzyl, cyanobenzyl, phenylethyl, phenylchloroethyl, isobutylbenzyl or phenyl optionally substituted by amino, $R^2$ denotes hydrogen, alkyl of 1 to 18 carbon atoms optionally substituted by piperonyl or pyrrolidinyl, or acetoxy, hydroxy or phenyl which in its turn is optionally substituted by alkyl of 4 carbon atoms, $R^2$ further denotes alkenyl of 9 carbon atoms, or imidazolyl, indanyl or phenyl which is monosubstituted by methoxy or hydroxy, or disubstituted by methyl, $R^3$ denotes hydrogen, alkyl of 1 to 4 carbon atoms, carboxyl, cyano or nitro, $R^4$ denotes hydrogen, alkyl of 1 to 3 carbon atoms, cyano, benzyl, nitro or cyclohexyl, and X denotes the anion of a mineral acid or lower fatty acid.

2. A fungicide containing a solid or liquid carrier and an imidazole-copper complex compound as defined in claim 1.

3. A process for the preparation of a fungicide as claimed in claim 2, *characterized in that* a solid or liquid carrier is mixed with an imidazole-copper complex compound as defined in claim 1.

4. A process for combatting fungi, *characterized in that* the fungi or the objects to be protected against fungus attack are treated with a fungicide as claimed in claim 1.

5. A process for the preparation of an imidazole-copper complex compound for a fungicide as claimed in claim 1, *characterized in that* an imidazole of the formula

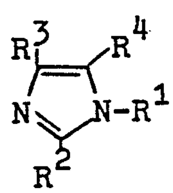

where $R^1$ to $R^4$ have the meanings given in claim 1, is reacted with a neutral or basic copper salt of a mineral acid or lower fatty acid.

6. A fungicide containing an imidazole-copper complex compound selected from the group consisting of 1-octylimidazole-$CuCl_2$, 1-dodecylimidazole-$CuCl_2$ and 1-tetradecylimidazole-$CuCl_2$.

**Revendications** (pour les Etats Contractants : BE, CH, DE, FR, GB, IT, LU, NL, SE)

1. Composés complexes cuivre-imidazole de formule

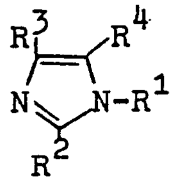

. $Cu(X)_2$

dans laquelle $R^1$ représente l'hydrogène, un reste alkyle en $C_1$ à $C_{18}$, qui est éventuellement substitué par morpholinyle, pipéridinyle, pipérazinyle, pyrrolidinyle, hydroxyle ou amino, qui à son tour est à nouveau, éventuellement, substitué par phényle et benzyle ou par deux restes alkyle en $C_1$ à $C_4$, $R^1$ représente en outre benzyle, cyanobenzyle, phényléthyle, phényl-chloréthyle, isobutylbenzyle ou un reste phényle, qui est, éventuellement, substitué par un groupe amino, $R^2$ représente l'hydrogène, un reste alkyle en $C_1$ à $C_{18}$, qui est, éventuellement, substitué par le reste pipéronyle ou pyrrolidine ou le reste acétoxy, ou le reste hydroxy ou le reste phényle qui est lui-même à nouveau éventuellement substitué par alkyle à 4 atomes de carbone. $R^2$ représente en outre un reste alcényle à 9 atomes de carbone, ou le reste imidazolyle ou le reste indanyle ou un reste phényle qui est substitué une fois par le reste méthoxy ou hydroxy, ou bien substitué deux fois par le reste méthyle, $R^3$ représente l'hydrogène, le reste alkyle en $C_1$ à $C_4$ ou le reste carboxyle, cyano ou nitro, et $R^4$ représente l'hydrogène, un reste alkyle en $C_1$ à $C_3$, le reste cyano, benzyle, nitro ou cyclohexyle, X représente l'anion d'un acide minéral ou un acide gras inférieur.

2. Fongicide contenant un composé complexe cuivre-imidazole selon la revendication 1.

3. Fongicide contenant un support solide ou liquide et un composé complexe cuivre-imidazole selon la revendication 1.

4. Procédé de préparation de fongicides, caractérisé par le fait qu'on mélange un matériau-support solide ou liquide avec un composé complexe de cuivre-imidazole selon la revendication 1.

5. Procédé de lutte contre les champignons, caractérisé par le fait que l'on traite les champignons, ou les objets à protéger contre les dégâts causés par les champignons, avec un composé complexe de cuivre-imidazole selon la revendication 1.

6. Procédé de préparation d'un composé complexe de cuivre-imidazole selon la revendication 1, caractérisé par le fait que l'on fait réagir un imidazole de formule

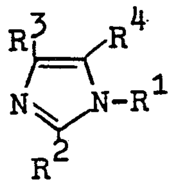

dans laquelle $R^1$ à $R^4$ ont les significations indiquées dans la revendication 1, avec un sel de cuivre neutre ou basique d'un acide minéral ou d'un acide gras inférieur.

7. Composés complexes de cuivre-imidazole choisis dans le groupe constitué par 1-octylimidazole-Cu-$Cl_2$, 1-Dodécylimidazole-$CuCl_2$, 1-tétradécylimidazole-$CuCl_2$.

8. Fongicide contenant un composé complexe de cuivre-imidazole choisi dans le groupe constitué par 1-octylimidazole-$CuCl_2$, 1-dodécylimidazole-$CuCl_2$, 1-tétradécylimidazole-$CuCl_2$.

**Revendications** (pour l'Etat Contractant : AT)

1. Fongicide contenant un composé complexe cuivre-imidazole de formule

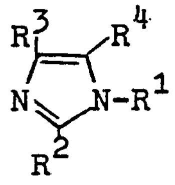

. $Cu(X)_2$

dans laquelle $R^1$ représente l'hydrogène, un reste alkyle en $C_1$ à $C_{18}$, qui est éventuellement substitué par morpholinyle, pipéridinyle, pipérazinyle, pyrrolidinyle, hydroxyle ou amino, qui à son tour est à nouveau, éventuellement, substitué par phényle et benzyle ou par deux restes alkyle en $C_1$ à $C_4$, $R^1$ représente en outre benzyle, cyanobenzyle, phényléthyle, phényl-chloréthyle, isobutylbenzyle ou un reste phényle, qui est, éventuellement, substitué par un groupe amino, $R^2$ représente l'hydrogène, un reste alkyle en $C_1$ à $C_{18}$, qui est, éventuellement, substitué par le reste pipéronyle ou pyrrolidine ou le reste acétoxy, ou le reste hydroxy ou le reste phényle qui est lui-même à nouveau éventuellement substitué par alkyle à 4 atomes de carbone, $R^2$ représente en outre un reste alcényle à 9 atomes de carbone, ou le reste imidazolyle ou le reste indanyle ou un reste phényle qui est substitué une fois par le reste méthoxy ou hydroxy, ou bien substitué deux fois par le reste méthyle, $R^3$ représente l'hydrogène, le reste alkyle en $C_1$ à $C_4$ ou le reste carboxyle, cyano ou nitro, et $R^4$ représente l'hydrogène, un reste alkyle en $C_1$ à $C_3$, le reste cyano, benzyle, nitro ou cyclohexyle, X représente l'anion d'un acide minéral ou un acide gras inférieur.

2. Fongicide contenant un support solide ou liquide et un composé complexe cuivre-imidazole selon la revendication 1.

3. Procédé de préparation d'un fongicide selon la revendication 2, caractérisé par le fait qu'on mélange un matériau-support solide ou liquide avec un composé complexe de cuivre-imidazole selon la revendication 1.

4. Procédé de lutte contre les champignons, caractérisé par le fait que l'on traite les champignons, ou les objets à protéger contre les dégâts causés par les champignons, avec un fongicide selon la revendication 1.

5. Procédé de préparation d'un composé complexe de cuivre-imidazole pour un fongicide selon la revendication 1, caractérisé par le fait que l'on fait réagir un imidazole de formule

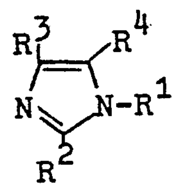

dans laquelle $R^1$ à $R^4$ ont les significations indiquées dans la revendication 1, avec un sel de cuivre neutre ou basique d'un acide minéral ou d'un acide gras inférieur.

6. Fongicide contenant un composé complexe de cuivre-imidazole choisi dans le groupe constitué par 1-octylimidazole-$CuCl_2$, 1-dodécylimidazole-$CuCl_2$, 1-tétradécylimidazole-$CuCl_2$.